(19) Europäisches Patentamt
European Patent Office
Office européen des brevets

(11) **EP 4 546 350 A1**

(12) **EUROPEAN PATENT APPLICATION**

(43) Date of publication:
**30.04.2025 Bulletin 2025/18**

(21) Application number: **24208114.9**

(22) Date of filing: **22.10.2024**

(51) International Patent Classification (IPC):
**G16B 15/30** $^{(2019.01)}$ **G06N 3/045** $^{(2023.01)}$
**G16B 40/20** $^{(2019.01)}$ **G16B 40/30** $^{(2019.01)}$

(52) Cooperative Patent Classification (CPC):
**G16B 15/30; G06N 3/045; G16B 40/20; G16B 40/30**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **23.10.2023 KR 20230142145**

(71) Applicant: **LG Management Development Institute Seoul 07336 (KR)**

(72) Inventors:
• **Hwang, Doyeong**
 **07524 Seoul (KR)**
• **Kim, Youjin**
 **07796 Seoul (KR)**
• **Kim, Kiyoung**
 **07811 Seoul (KR)**
• **Yim, Soorin**
 **07801 Seoul (KR)**

(74) Representative: **BCKIP Part mbB**
 **MK1**
 **Landsbergerstraße 98, 3.Stock**
 **80339 München (DE)**

(54) **BONDING PREDICTION DEVICE FOR PREDICTING MHC-PEPTIDE COMPLEX PROPERTY BASED ON ARTIFICIAL INTELLIGENCE AND METHOD USING THE SAME**

(57) A bonding prediction device for predicting MHC-peptide complex activity based on artificial intelligence comprises: memory; a communication unit; and at least one processor operably connected to the memory and the communication unit, wherein the at least one processor may be configured to: obtain data related to a structure of a major histocompatibility complex(MHC)-peptide complex, which comprises a first sub-unit and a second sub-unit, from the memory; extract first coordinate information, which is 3D coordinates of the first subunit; extract second coordinate information, which is 3D coordinates of the second subunit; calculate distance information between the first sub-unit and the second sub-unit based on the first coordinate information and the second coordinate information; and perform learning using an artificial intelligence model to predict bonding of the MHC-peptide complex based on the distance information.

FIG. 4

## Description

## BACKGROUND

The present disclosure generally relates to a bonding prediction device for predicting a property of a major histocompatibility complex (MHC)-peptide complex based on artificial intelligence and a method using the same. More specifically, some embodiments of the present disclosure may provide prediction results for predicting a property of an MHC-peptide complex by using a distance between sub-units comprised in a protein complex MHC-peptide as input for learning via artificial intelligence.

New types of antigens composed of protein complexes may be derived from somatic mutations, formed by target cancer cells and antigen-presenting cells. Immunogenicity refers to the activation of T cells that recognize a protein complex where MHC proteins and peptide antigens are bound. For the T cells to induce immunogenicity, T-cell receptors need to be physically bound to the protein complex composed of MHC proteins and peptide antigens.

Recently, the performance of various learning models for predicting the unknown structure of MHC-peptide complexes has been developed. However, due to noise or high cost that may occur during the learning process, it is challenging to directly use the MHC-peptide complex structure in bond prediction.

Therefore, a learning approach which can more accurately predict the property of a complex based on an input value of the MHC-peptide complex may be needed.

Particularly, a learning approach that enhances prediction performance by utilizing complex structural information such as protein-protein, protein-peptide, and protein-ligand may be needed to predict information related to the property of MHC-peptide complexes.

[Related Art Document]

[Patent Document]

Korean Patent Application Publication No. 10-2022-0064958.

## SUMMARY

Some embodiments of the present disclosure may provide immunogenicity data corresponding to a property of an MHC-peptide complex by extracting a distance between sub-units of the MHC-peptide complex based on information related to a structure of the MHC-peptide complex.

According to certain embodiments of the present disclosure, a bonding prediction device may provide accurate data on residue alpha carbons by searching for a 3D structure of possible MHC-peptide complexes through iterative learning.

The problems solved by the present disclosure are not limited to those mentioned above, and other problems not mentioned may be clearly understood by those skilled in the art from the description below.

A bonding prediction device for predicting MHC-peptide complex activity based on artificial intelligence according to an embodiment of the present disclosure may comprising: a memory; a communication unit; and at least one processor electrically connected to the memory and the communication unit, wherein the at least one processor may be configured to: obtain structure data of an MHC-peptide complex, which consists of at least a first subunit and a second subunit, from the memory; extract first coordinate information, which is the 3D coordinates of the first subunit; extract second coordinate information, which is the 3D coordinates of the second subunit; calculate distance information between the first subunit and the second subunit based on the first coordinate information and the second coordinate information; and perform learning using an artificial intelligence model to predict the bonding of the MHC-peptide complex based on the distance information.

Abonding prediction method for predicting MHC-peptide complex property based on artificial intelligence, performed by at least one processor of a computer device may comprise: obtaining structure data of an MHC-peptide complex, which consists of at least a first subunit and a second subunit; extracting first coordinate information, which is the 3D coordinates of the first subunit; extracting second coordinate information, which is the 3D coordinates of the second subunit; calculating distance information between the first subunit and the second subunit based on the first coordinate information and the second coordinate information; and predicting the bonding of the MHC-peptide complex based on the distance information using a learned artificial intelligence model.

In addition, other methods and systems for implementing certain embodiments of the present disclosure and a computer-readable recording medium storing a computer program for executing the method, may further be provided.

Furthermore, a computer program stored on a medium that allows the method of implementing the present disclosure to be performed on a computer may further be provided.

According to certain embodiments of the present disclosure, a bonding prediction device for predicting a property of an MHC-peptide complex based on artificial intelligence can extract the property of the MHC-peptide complex, capable of being physically bound with a T-cell receptor, based on distance information between sub-units of the MHC-peptide complex using protein sequences.

The effects of the present disclosure are not limited to those mentioned above, and other effects not mentioned may be clearly understood by those skilled in the art from the description below.

**BRIEF DESCRIPTION OF DRAWINGS**

FIG. 1 is a schematic block diagram of a binding prediction device that predicts major histocompatibility complex(MHC)-peptide complex activity based on artificial intelligence according to various embodiments of the present disclosure.

FIG. 2 is a conceptual diagram for illustrating a process for binding prediction for determining immunogenicity by a binding prediction device configured to predict MHC-peptide complex activity based on artificial intelligence according to various embodiments of the present disclosure.

FIG. 3 is a conceptual diagram for illustrating exchange information between a pair rep module and a cross operation module of a binding prediction device configured to predict MHC-peptide complex activity based on artificial intelligence according to various embodiments of the present disclosure.

FIG. 4 illustrates an example of acquiring MHC and interpeptide distance information of an MHC-peptide complex according to various embodiments of the present disclosure.

FIG. 5 is a diagram for illustrating an exemplary process of obtaining distance information between sub-units of an MHC-peptide complex according to various embodiments of the present disclosure.

FIG. 6 is a schematic flowchart of a binding prediction method for predicting MHC-peptide complex activity based on artificial intelligence according to various embodiments of the present disclosure.

FIG. 7 is an overall flowchart of an immunogenicity prediction method for predicting MHC-peptide complex activity based on artificial intelligence according to various embodiments of the present disclosure.

FIG. 8 is a schematic flowchart for illustrating cross attention calculation of a binding prediction method for predicting MHC-peptide complex activity based on artificial intelligence according to various embodiments of the present disclosure.

FIG. 9 is a schematic flowchart for illustrating a pair representation update of a binding prediction method for predicting MHC-peptide complex activity based on artificial intelligence according to various embodiments of the present disclosure.

FIG. 10 is a conceptual diagram for illustrating a binding prediction method for predicting MHC-peptide complex activity based on artificial intelligence to output immunogenicity (IMM) according to binding according to various embodiments of the present disclosure.

**DETAILED DESCIPTION**

Throughout the present disclosure, the same reference numerals designate the same components. The present disclosure does not describe all elements of the embodiments, and general contents in the technical field of the present disclosure or duplicated content among the embodiments are omitted. The terms "unit, module, member, block" used in the specification may be implemented in software or hardware, and depending on the embodiments, multiple "units, modules, members, blocks" may be implemented as a single component, or a single "unit, module, member, block" may also include multiple components.

Throughout the specification, when a part is described as being "connected" to another part, it includes not only cases where they are directly connected but also cases where they are indirectly connected, which may connected by a wireless communication network.

Furthermore, when a part is described as "comprising" a certain component, it means that it may further include other components, not excluding other components unless explicitly stated otherwise.

Throughout the specification, when one member is described as being "on" other member, it includes not only cases where the members are in contact but also cases where another member exists between them.

Terms such as "first" and "second" are used to distinguish one component from another, and are not intended to limit the components to those aforementioned by the terms.

Singular expressions include plural expressions unless the context clearly indicates otherwise.

Identification codes used for each step are provided for convenience in description and do not specify the order of the steps, and each step may be carried out in a different order unless a specific order is explicitly described.

The operating principles and embodiments of the present disclosure will be described below with reference to the accompanying drawings.

The term "device according to the present disclosure" in the present disclosure encompasses various devices capable of performing operations and providing results to users. For example, the device according to the present disclosure may include a computer, server device, and portable terminal, or it may take any one of these forms.

Here, the computer may include, for example, a notebook, desktop, laptop, tablet PC, or slate PC, equipped with a web browser.

The server device is a server that processes information by communicating with an external device, and may include an application server, a computing server, a database server, a file server, a game server, a mail server, a proxy server, and a web server.

The portable terminal is for example, a wireless communication device ensuring portability and mobility, and may include all kinds of handheld-based wireless communication devices such as Personal Communication System (PCS), Global System for Mobile communications (GSM), Personal Digital Cellular (PDC), Personal Handyphone System (PHS), Personal Digital Assistant (PDA), International Mobile Telecommunication (IMT)-2000, Code Division Multiple Access (CDMA)-2000, W-Code Division Multiple Access (W-CDMA), Wireless Broadband Internet (WiBro) terminal, smart phone, and wearable devices such as watches, rings, bracelets, anklets, necklaces, glasses, contact lenses, or head-mounted devices (HMD).

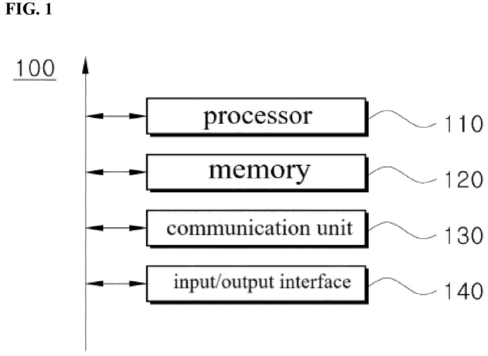

FIG. 1 is a schematic block diagram of a binding prediction device 100 for predicting major histocompatibility complex(MHC)-peptide complex activity based on artificial intelligence according to various embodiments of the present disclosure.

A bonding prediction device 100 may include one or more processors 110, memory 120, a communicator or communication unit 130, and an input and/or output interface 140, and the like. However, these internal components included in the bonding prediction device 100 are provided for illustration purposes only, although not limited thereto. Alternatively or additionally, the bonding prediction device 100 according to an embodiment of the present disclosure may perform the functions of the processor 110 through a separate processing server or a cloud server instead of a processor.

Referring to FIG. 1, the processor 110 may be configured to perform one or more operations of the bonding prediction device 100 in association with the memory 120 that stores data on an algorithm for controlling the operation of components included in or associated with the bonding prediction device 100 or a program that reproduces the algorithm, and the data stored in the memory 120. For example, the processor 110 and memory 120 may be implemented as separate chips. Alternatively, the processor 110 and the memory 120 may be implemented as an integrated single chip.

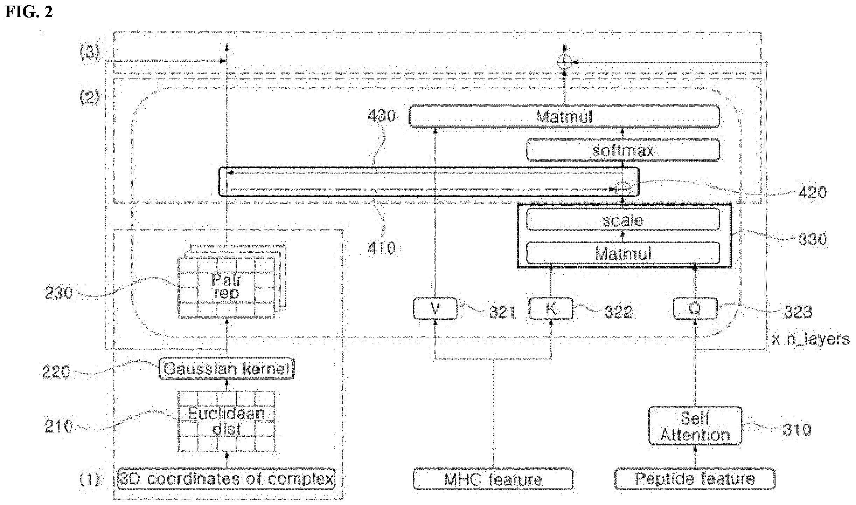

The processor 110 may control one or more of the components described above to implement various embodiments according to the present disclosure in the bonding prediction device 100, which are described in FIGS. 2 to 8.

The memory 120 according to an embodiment may store data performing or supporting various functions of the bonding prediction device 100, programs for the operation of the processor 110, input and/or output data (e.g., images, videos, etc.), multiple application programs or applications running on the bonding prediction device 100, and data or instructions for operating the bonding prediction device 100. At least some of these application programs may be downloaded from an external server via wired or wireless communication.

The memory 120 may include at least one type of storage medium, such as flash memory type, hard disk type, Solid State Disk (SSD) type, Silicon Disk Drive (SDD) type, multimedia card micro type, card type memory (e.g., SD or XD memory), random access memory (RAM), static random access memory (SRAM), read-only memory (ROM), electrically erasable programmable read-only memory (EEPROM), programmable read-only memory (PROM), magnetic memory, magnetic disk, and optical disk. Additionally, the memory may be a database that is separate from the bonding prediction device 100, but may be connected wired or wirelessly.

The communicator or communication unit 130 according to an embodiment may include one or more components configured to communicate with external devices, including, for example, at least one of a broadcast receiving module or broadcast receiver, wired communication module or wired communicator, wireless communication module or wireless communicator, short-range communication module or short-range communicator, or location information module.

The wired communication module may include various wired communication modules such as a Local Area Network (LAN) module, a Wide Area Network (WAN) module, or a Value Added Network (VAN) module, as well as various cable communication modules such as Universal Serial Bus (USB), High Definition Multimedia Interface (HDMI), Digital Visual Interface (DVI), recommended standard 232 (RS-232), power line communication, or plain old telephone service (POTS).

The wireless communication module may include not only a WiFi module, a Wireless broadband (WiBro) module, but also a wireless communication module supporting various wireless communication methods such as Global System for Mobile Communication (GSM), Code Division Multiple Access (CDMA), Wideband Code Division Multiple Access (WCDMA), Universal Mobile Telecommunications System (UMTS), Time Division Multiple Access (TDMA), Long Term Evolution (LTE), 4G (Generation), 5G, and 6G.

The short-range communication module is for short-range communication and may support short-range communication using at least one of the following technologies: Bluetooth™, Radio Frequency Identification (RFID), Infrared Data Association (IrDA), Ultra Wideband (UWB), ZigBee, Near Field Communication (NFC), Wireless-Fidelity (Wi-Fi), Wi-Fi Direct, or Wireless Universal Serial Bus (Wireless USB).

The input and/or output interface 140 according to an embodiment serves as a channel for connecting various types of external devices to the bonding prediction device 100. The input and/or output interface 140 may include, for example, but not limited to, at least one of the following: a wired and/or wireless headset port, an external charger port, a wired and/or wireless data port, a memory card port, a port for connecting a device equipped with a Subscriber Identification Module (SIM), an audio Input/Output (I/O) port, a video Input/Output (I/O) port, or an earphone port. The bonding prediction device 100 may perform control related to the external device connected to the input and/or output interface 140.

At least one component may be added or omitted in accordance with the performance of the components shown in FIG. 1. It will be readily understood by those skilled in the art that the mutual positions of the components may also be modified in accordance with the performance or structure of the device.

Meanwhile, each component shown in FIG. 1 represents software and/or hardware components such as a Field Programmable Gate Array (FPGA) and an Application Specific Integrated Circuit (ASIC).

FIG. 2 is a conceptual diagram for illustrating a bonding prediction operational process for predicting immunogenicity according to various embodiments of the present disclosure. The bonding prediction operation process may be performed by the processor 110 of the bonding prediction device 100 of FIG. 1. Hereinafter, the invention will be described with reference to Figures 2 through 10.

Referring to Figure 2, at least one processor 110 according to one embodiment of the present disclosure calculates an Euclidean distance between sub-units of a major histocompatibility complex(MHC)-peptide complex from the MHC-peptide complex's 3D coordinates by a Euclidean distance calculation module 210 based on pre-determined operations, and outputs pair representation corresponding to amino acid arrangement of a protein complex based on the Euclidean distance by a Gaussian kernel 220. A pair-rep module 230 may be a module configured to output the pair representation. Hereinafter, the pair representation is referred to as pair-rep as shown in (1) of FIG. 2.

The MHC-peptide complex may comprise a first sub-unit and a second sub-unit. First coordinate information of the first sub-unit may comprise 3D coordinates of the first sub-unit and second coordinate information of the second sub-unit may comprise 3D coordinates of the second sub-unit.

As an example, the first sub-unit may be a peptide, and the second sub-unit may be an MHC. Hereinafter, some embodiments of the present disclosure will be explained using an example where the first sub-unit is a peptide and the second sub-unit is an MHC for illustration purposes only, although the present disclosure is not limited thereto.

Referring to FIG. 2, an Euclidean distance ($d_{ij}$) between the peptide and MHC pair may be calculated based on 3D coordinate information of the MHC-peptide complex, which includes the first coordinate information of the first sub-unit and the second coordinate information of the second sub-unit.

The Gaussian kernel 220 has trainable parameters $\mu$ and $\sigma$ as part of a Gaussian density function, which may be performed using Equation (1).

$$g(d, \mu, \sigma) = \frac{1}{\sigma\sqrt{2\pi}} e^{-\frac{(d-\mu)^2}{2\sigma^2}} \ldots\ldots \text{Equation (1)}$$

Thus, the processor 110 can obtain the pair-rep($p_{ij}$) by using the Euclidean distance ($d_{ij}$) through the Gaussian kernel 220, as in Equation (2).

$$p_{ij} = \left\{ g(d_{ij}, \mu^k, \sigma^k) \middle| k \in [1, D] \right\} \ldots \text{Equation (2)}$$

In other words, the Euclidean distance calculated from the 3D coordinates of the MHC-peptide complex is used to output an initial pair-rep by the trainable Gaussian kernel 220. Since the initial pair-rep can have the same form as the multi-head cross-attention of the MHC features and peptide features, the initial pair-rep may be summed with an output of a cross-attention operation of a cross-attention module 330 as a bias term.

Referring to FIG. 2, a self-attention module 310 may perform a self-attention operation using a peptide feature extracted based on at least one protein sequence, and extract an MHC embedding that was embedded using a pre-learned model based on the MHC feature extracted from at least one protein sequence.

The output of performing the self-attention operation may be input as a query (Q: a query 323), and an MHC embedding vector may be input as a key (K: a key 322) and a value (V: a value 321). Additionally, the cross-attention module 330 can perform a cross-attention operation based on the input key 322 and query (323), as shown in (2) of FIG. 2.

Specifically, represents the projection of the channel dimension of pair-rep ($p_{ij}$) to match the number of heads of the cross-attention module 330 to enable addition to the cross multi-head attention (MHA). This can be obtained through Equation (3).

$$q_{ij}^0 = p_{ij}M, q_{ij}^{l+1} = q_{ij}^l + \left\{ \frac{Q_i^{l,h}(K_j^{l,h})^T}{\sqrt{d}} \middle| h \in [1,H] \right\} ...\text{Equation (3)}$$

In one embodiment, $q_{ij}$ may be updated by being passed through n layers.

Then, the pair-rep module 230 and the cross-attention module 330 can exchange the initial pair-rep of the pair-rep module 230 and the output of the cross-attention operation of the cross-attention module 330 (410, 430).

Referring to FIG. 2, after receiving the initial pair-rep (410), a sum operation (420) may be performed on the initial pair-rep as a bias term for the output of the cross-attention operation using the peptide feature and the MHC embedding. An operation result can be obtained using Equation (4), where the left side of Equation (4) is an attention score, a left term in the right side of Equation (4) is a cross-attention result, and a right term in the right side of Equation (4) is a bias term.

$$\text{Attention}(Q_i^{l,h}, K_j^{l,h}, V_j^{l,h}) = \text{softmax}(\frac{Q_i^{l,h}(K_j^{l,h})^T}{\sqrt{d}} + q_{ij}^{l-1,h})V_j^{l,h} ...\text{Equation (4)}$$

Therefore, the attention score is defined as the result of adding pair-rep as a bias term to the output of the cross-attention operation of the cross-attention module 330 followed by applying softmax.

Additionally, an output of summing the pair representation as a bias term with the output of the cross-attention operation is received (430), and the pair-rep may be updated using the received output.

The bonding prediction device 100 according to an embodiment of the present disclosure can predict the bonding corresponding to the MHC-peptide complex using either the output of summing the pair representation as the bias term with the output of the cross-attention operation or the updated pair-rep, thereby obtaining immunogenicity data, as shown in (3) of FIG. 2.

In other words, using the pair-rep and the embedding vector, the bonding prediction device 100 can predict the structure of the MHC-peptide complex and determine the bonding corresponding to the MHC-peptide complex.

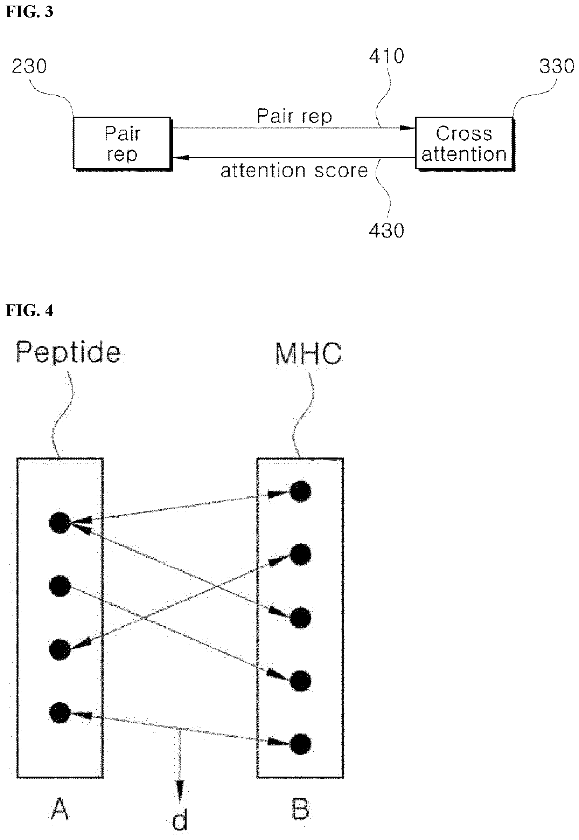

Specifically, referring to FIG 3, the pair-rep module 230 and the cross-attention module 330 can exchange either the pair-rep (410) or the attention score (430). The pair-rep (410) can be the initial pair-rep output from the pair-rep module 230. The attention score (430) can be the output of summing the pair representation as a bias term with the output of the cross-attention operation.

The bonding prediction device 100 according to an embodiment of the present disclosure may acquire data related to a structure of an MHC-peptide complex comprising a first sub-unit and a second sub-unit, extract first coordinate information, which includes the 3D coordinate of the first sub-unit, extract second coordinate information, which includes the 3D coordinate of the second subunit, and calculate a distance between the first sub-unit and the second sub-unit based on the first and second coordinate information. The bonding prediction device 100 may be configured to learn an artificial intelligence model to predict the binding of the MHC-peptide complex based on the distance between the first sub-unit and the second sub-unit.

As illustrated in FIG. 4, amino acids corresponding to a peptide (A) constituting the MHC-peptide complex are arranged in one block, while amino acids corresponding to an MHC (B) are arranged in the other block. At this time, the distance (d) between the amino acid of the peptide (A) and the amino acid of the MHC (B) can be acquired. Using the distance (d) between the amino acid of the peptide (A) and the amino acid of the MHC (B), the binding of the MHC-peptide complex composed of peptide (A) and MHC (B) can be predicted to determine immunogenicity.

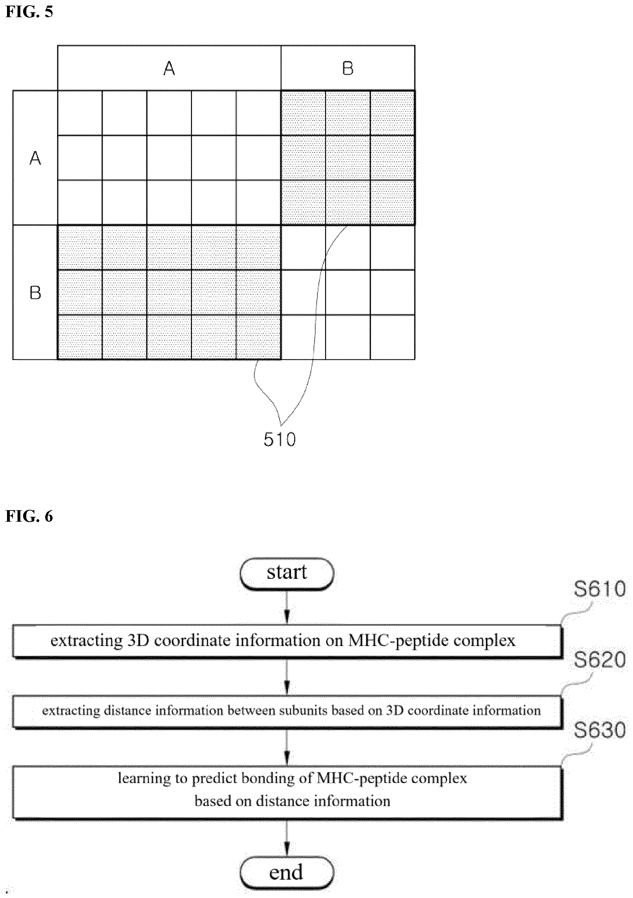

As illustrated in FIG. 5, the distances between the amino acids of the peptide (A) or the distance between the amino acids of MHC (B) are not used. Only the distance between the amino acid of the peptide (A) and the amino acid of the MHC (B) is used. Using the distance (510) between the amino acid of the peptide (A) and the amino acid of the MHC (B) arranged in a rep-pair, the structure and binding of the MHC-peptide complex can be predicted to determine immunogenicity.

As illustrated in FIG. 6, in step S610, 3D coordinate information on the MHC-peptide complex can be extracted. For example, in step S610, the processor 110 can use information on a structure of the MHC-peptide complex, such as the types of sub-units and the amino acid arrangement information of the sub-units constituting the MHC-peptide complex. The 3D coordinate values of the amino acids constituting the sub-units of the MHC-peptide complex can be obtained.

In step S620, a distance between sub-units may be extracted based on 3D coordinate information. The processor 110 can acquire the distances between amino acids of different sub-units using the 3D coordinate values.

In step S630, the processor 110 may be learned to predict the structure and binding of the MHC-peptide complex based on the distance between the sub-units. By utilizing the distance between amino acids of different sub-units, the processor 110 can learn an artificial intelligence model to predict whether the MHC-peptide complex having the sub-units can be physically bound to a T-cell receptor and activate T cells to elicit immunogenicity.

In step S610, to extract the 3D coordinate information, the processor 110 can verify data on at least one protein sequence from the memory 120.

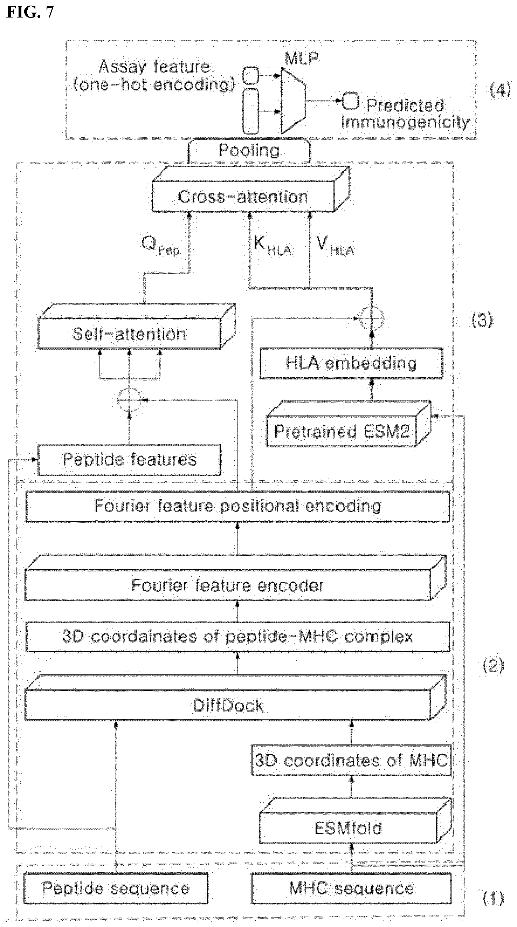

For example, as illustrated in FIG. 7, at least one protein sequence may include an MHC sequence and a peptide sequence. The processor 110 can determine whether the structure of the MHC-peptide complex composed of the MHC sequence and the peptide sequence can be recognized as a specific antigen by a T cell and activate the T cell.

According to an embodiment, the processor 110 can predict a first protein complex based on data related to at least one protein sequence. For example, the first protein complex may include the MHC-peptide complex. Specifically, the processor 110 can provide immunogenicity data by predicting whether the MHC-peptide complex can be recognized as a specific antigen and activate T cells based on the learned data related to distances between sub-units of the MHC-peptide complex.

According to an embodiment, the processor 110 can extract the 3D coordinates of the MHC based on the MHC sequence using a first model. The first model can be, for example, but not limited to, an ESMfold model. The processor 110 can input the MHC sequence into the first model to predict the 3D coordinates (e.g., a 3D structure) of the MHC. In other words, the processor 110 can extract the 3D coordinates of the MHC through a learned model based on the MHC sequence.

According to an embodiment, the processor 110 can extract the 3D coordinates of an MHC-peptide complex based on the 3D coordinates of the MHC and the peptide sequence using a second model. The second model may be, for instance, but not limited to, a DiffDock model. The processor 110 can input the 3D coordinates of the MHC and the peptide sequence into the second model to predict the 3D coordinates (e.g., a 3D structure) of the MHC-peptide complex. In other words, the processor 110 can extract the 3D coordinates of the MHC-peptide complex through a learned model based on the MHC coordinates and the peptide sequence.

According to an embodiment, the processor 110 can compute Fourier feature positional encoding from the 3D coordinates of the MHC-peptide complex based on predetermined operations. For instance, the processor 110 can utilize a Fourier feature encoder model. The processor 110 can calculate the Fourier feature positional encoding by using a residue-level 3D structure of the MHC-peptide complex as input. The Fourier feature positional encoding can be performed using Equations (5) to (7).

$$F \leftarrow \frac{1}{\sqrt{|F|}} [\cos X W_r^T \,; \sin X W_r^T]...\text{Equation (5)}$$

$$Y \leftarrow GeLU(FW_1 + B_1)W_2 + B_2...\text{Equation (6)}$$

$$PE_X \leftarrow \text{Reshape Y into the shape of} [N, D];...\text{Equation (7)}$$

In Equations (5) to (7), $X \in R^{N \times |F|}$ represents 3D coordinate data x of each residue alpha carbon (M=3). According to an embodiment, the processor 110 can compute the Fourier feature positional embedding to extract PEx.

$W_r \in R^{\frac{|F|}{2} \times M}$, $W_1 \in R^{|F| \times |H|}$, $W_2 \in R^{|H| \times |D|}$ is a trainable weight, and $B_1 \in R^{|H|}$ and $B_2 \in R^{|D|}$ are trainable biases. Additionally, $|F|$, $|H|$, $|D|$ represent input feature, hidden, and output dimensions, respectively, N is a sequence length, and "" denotes a concatenation operation.

According to an embodiment, the processor 110 can perform self-attention by summing the peptide feature extracted based on the peptide sequence and the positional encoding. The processor 110 can extract the MHC embedding (MHC Embedding) using a pre-learned model based on the MHC sequence. For example, the pre-learned model may be ESM2. The processor 110 can perform cross-attention using an output of summing the positional encoding and the MHC embedding with the output of performing the self-attention. During the positional encoding, the processor 110 can apply individual weights to the 3D coordinates of each residue alpha carbon.

A process (4) of FIG. 7 may correspond to the process (3) in FIG. 2, and by inputting the output of performing the cross-attention into a MLP (Multi-Layer Perceptron) neural network, the immunogenicity (IMM) predicted through the MHC-peptide complex structure can be output.

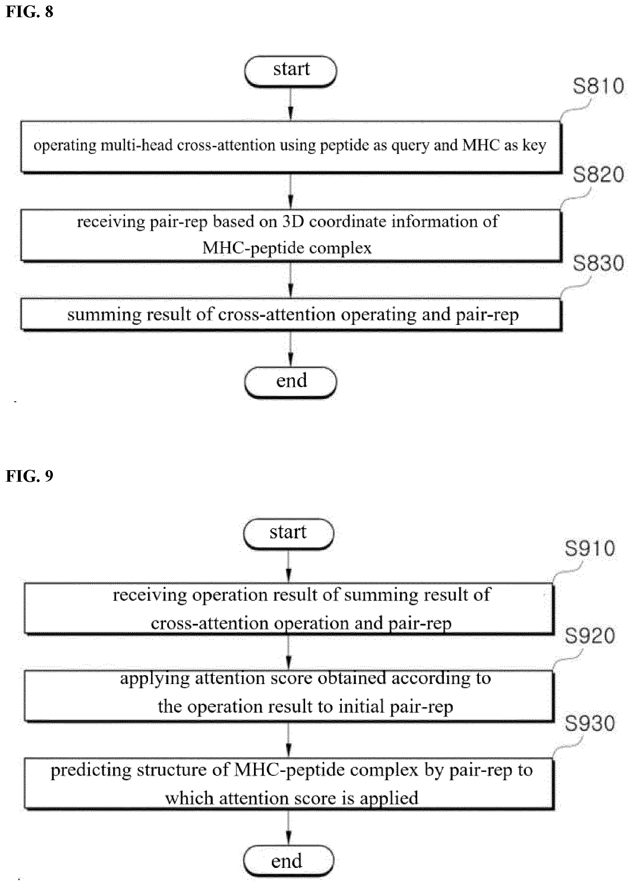

Additionally, when performing the cross-attention operation, in the cross-attention module 330, as illustrated in FIG. 8, in step S810, the peptide is used as a query (Q), and the MHC as a key (K) to perform multi-head cross-attention.

Furthermore, in step S820, based on the 3D coordinate information of the MHC-peptide complex, pair-rep can be received. The pair-rep generated from the pair-rep module 230 can be input to the cross-attention module 330.

In step S830, the output of performing cross-attention result and the pair-rep can be summed.

In an embodiment, the output of performing the self-attention can be used as a query, and the output of summing the positional encoding and the HLA embedding can be used as a key and a value to perform an attention operation to predict the binding of the MHC-peptide complex.

In this embodiment, the peptide feature can be used as a query, and the MHC feature as a key to perform cross-attention and obtain an embedding vector.

Meanwhile, as shown in FIG. 9, in step S910, an output of summing the output of performing the cross-attention operation with the pair-rep is received by the pair-rep module 230.

In step S920, the attention score obtained based on the output of summing the output of performing the cross-attention operation with the pair-rep can be applied to the initial pair-rep.

In step S930, using the attention score applied to the pair-rep, the structure of the MHC-peptide complex can be predicted to determine the immunogenicity.

Redundant details described above have been omitted to simplify the description of some embodiments of the present disclosure.

Therefore, according to an embodiment of the present disclosure, a bonding prediction device 100 for predicting MHC-peptide complex property based on artificial intelligence includes memory 120, a communication unit or communicator 130, and at least one processor 110 operably connected to the memory 120 and the communication unit communicator 130. The processor 110 may acquire data related to a structure of an MHC-peptide complex comprising a first sub-unit and a second sub-unit from the memory 120, extract first coordinate information, which is 3D coordinate information of the first sub-unit and second coordinate information, which is 3D coordinate information of the second sub-unit, calculate distance information between the first sub-unit and the second sub-unit based on the first coordinate information and the second coordinate information, and perform learning using an artificial intelligence model to predict the binding of the MHC-peptide complex based on the distance information.

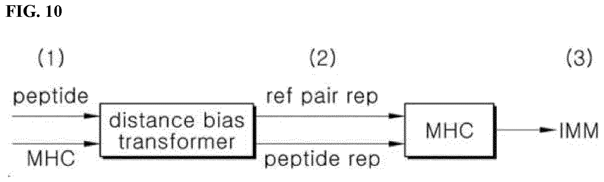

As shown in FIG. 10, information related to a structure of a protein complex related to a first sub-unit (e.g. peptide) and a second sub-unit (e.g. MHC) is obtained (operation (1) of FIG. 10). After inputting the first sub-unit and the second sub-unit into a distance bias transformer, a final pair-rep (e.g. ref pair-rep) and peptide representation (e.g. peptide rep) are obtained at operation (2) of FIG. 10. The ref pair-rep may be a value obtained by applying the attention score to the initial pair-rep, and the peptide rep may be obtained by adding the initial pair-rep to the output of performing the cross-attention operation using the peptide feature and the MHC feature.

According to some embodiments of the present disclosure, the bonding prediction device 100 can predict immunogenicity (IMM) through by an MLP neural network having a pooling layer that receives the ref pair-rep and the peptide rep as inputs at operation (3) of FIG. 10.

Meanwhile, certain embodiments of the present disclosure may be implemented in the form of a recording medium that stores instructions executable by a computer. The instructions may be stored in the form of program code and, when executed by a processor, may generate program modules to perform the operations of the disclosed embodiments. The recording medium may be implemented as a computer-readable recording medium.

The computer-readable recording medium includes any type of recording medium in which instructions that may be decrypted by a computer are stored. For example, Examples include a read only memory (ROM), a random access memory (RAM), a magnetic tape, a magnetic disk, a flash memory, an optical data storage device, and the like.

As described above, the disclosed embodiments are described with reference to the accompanying figures. A person of ordinary skill in the art may understand that the present disclosure may be implemented in a different form from the disclosed embodiments without changing the technical sprit or essential features of the present disclosure. The disclosed embodiments are illustrative and restrictive.

**[Explanation of Symbols]**

100: immunogenicity prediction device
110: processor
120: memory
130: communication unit
140: input/output interface
210: Euclidean distance calculation module
220: Gaussian Kernel

230: pair-rep module
310: self-attention operation module
321: value
322: key
323: query
330: cross-attention operation module

**Claims**

1. A system comprising:

   memory; and
   at least one processor operably connected to the memory, the at least one processor configured to:

   obtain data related to a structure of a major histocompatibility complex(MHC)-peptide complex, which comprises a first sub-unit and a second sub-unit, from the memory;
   extract 3D coordinates of the first sub-unit;
   extract 3D coordinates of the second sub-unit;
   calculate a distance between the first sub-unit and the second sub-unit based on the 3D coordinates of the first sub-unit and the 3D coordinates of the second sub-unit; and
   perform learning using an artificial intelligence model to predict bonding of the MHC-peptide complex based on the distance between the first sub-unit and the second sub-unit.

2. The system according to claim 1, wherein:

   the first sub-unit comprises a peptide, and
   the second sub-unit is an MHC.

3. The system according to claim 2, wherein:
   the at least one processor is configured to:

   calculate an Euclidean distance between the first sub-unit and the second sub-unit from the 3D coordinates of the first sub-unit and the 3D coordinates of the second sub-unit of the MHC-peptide complex based on a pre-determined operation; and
   output a pair representation corresponding to amino acid placement of the MHC-peptide complex using a Gaussian kernel based on the Euclidean distance between the first sub-unit and the second sub-unit.

4. The system according to claim 3, wherein:
   the at least one processor is configured to:

   receive the pair representation,
   obtain an output of summing the received pair representation as a bias term with an output of performing cross-attention using a peptide feature and MHC embedding, and
   output immunogenicity data corresponding to the MHC-peptide complex based on the output of summing the received pair representation as the bias term with the output of performing the cross-attention.

5. The system according to claim 4, wherein the at least one processor is configured to:

   update the pair representation based on the output of summing the received pair representation as the bias term with the output of performing the cross-attention, and
   output the immunogenicity data corresponding to the structure of the MHC-peptide complex based on the updated pair representation.

6. The system according to claim 4, wherein the at least one processor is configured to:

   calculate positional encoding from the 3D coordinates of the first sub-unit and the 3D coordinates of the second sub-unit of the MHC-peptide complex based on a predetermined operation,

perform self-attention by summing the peptide feature, which is extracted based on at least one protein sequence, with the positional encoding,

extract the MHC embedding, which has been embedded using a pre-learned model according to an MHC feature extracted based on the at least one protein sequence, and

perform the cross-attention with an output of summing the positional encoding, the MHC embedding, and an output of performing the self-attention.

7. The system according to claim 6, wherein the at least one processor is configured to extract the 3D coordinates of the first sub-unit and the 3D coordinates of the second sub-unit of the MHC-peptide complex using DiffDock and ESMfold models.

8. The system according to claim 6, wherein the at least one processor is configured to predict the structure of the MHC-peptide complex based on an embedding vector obtained by an attention operation by inputting the output of the self-attention as a query, inputting an output of summing the positional encoding and the MHC embedding as a key and value.

9. The system according to claim 8, wherein the at least one processor is configured to obtain the embedding vector by a cross-attention operation by inputting the peptide feature as a query and the MHC feature as a key.

10. A computer-implemented method comprising:

obtaining data related to a structure of a major histocompatibility complex(MHC)-peptide complex, which comprises a first sub-unit and a second sub-unit;
extracting 3D coordinates of the first sub-unit;
extracting 3D coordinates of the second sub-unit;
calculating a distance between the first sub-unit and the second sub-unit based on the 3D coordinates of the first sub-unit and the 3D coordinates of the second sub-unit; and
predicting bonding of the MHC-peptide complex based on the distance between the first sub-unit and the second sub-unit using a learned artificial intelligence model.

11. The computer-implemented method according to claim 10, wherein:

the first sub-unit comprises a peptide,
the second sub-unit is an MHC, and
the at least one processor:

calculates an Euclidean distance between the first sub-unit and the second sub-unit from the 3D coordinates of the first sub-unit and the 3D coordinates of the second sub-unit of the MHC-peptide complex based on a predetermined operation; and
outputs a pair representation corresponding to amino acid placement of the MHC-peptide complex using a Gaussian kernel based on the Euclidean distance between the first sub-unit and the second sub-unit.

12. The computer-implemented method according to claim 11, wherein:
the at least one processor:

receives the pair representation,
obtains an output of summing the received pair representation as a bias term with an output of performing cross-attention using a peptide feature and MHC embedding, and
outputs immunogenicity data corresponding to the MHC-peptide complex based on the output of summing the received pair representation as the bias term with the output of performing the cross-attention.

13. The computer-implemented method according to claim 12, wherein:
the at least one processor:

updates the pair representation based on the output of summing the received pair representation as the bias term with the output of performing the cross-attention, and
outputs the immunogenicity data corresponding to the structure of the MHC-peptide complex based on the updated pair representation.

**14.** The computer-implemented method according to claim 12, wherein:
the at least one processor:

calculates positional encoding from the 3D coordinates of the first sub-unit and the 3D coordinates of the second sub-unit of the MHC-peptide complex based on a predetermined operation,
performs self-attention by summing the peptide feature, which is extracted based on at least one protein sequence, with the positional encoding,
extracts the MHC embedding, which has been embedded using a pre-learned model according to an MHC feature extracted based on the at least one protein sequence, and
performs the cross-attention with an output of summing the positional encoding, and the MHC embedding, and an output of performing the self-attention.

**15.** The computer-implemented method according to claim 14, wherein:
the at least one processor predicts the structure of the MHC-peptide complex based on an embedding vector obtained by an attention operation by inputting the output of the self-attention as a query, inputting an output of summing the positional encoding and the MHC embedding as a key and value.

**16.** A non-transitory computer-readable storage medium having instructions that, when executed by one or more processors, cause the one or more processors to:

obtain data related to a structure of a major histocompatibility complex(MHC)-peptide complex, which comprises a first sub-unit and a second sub-unit;
extract 3D coordinates of the first sub-unit;
extract 3D coordinates of the second sub-unit;
calculate a distance between the first sub-unit and the second sub-unit based on the 3D coordinates of the first sub-unit and the 3D coordinates of the second sub-unit; and
predict bonding of the MHC-peptide complex based on the distance between the first sub-unit and the second sub-unit using a learned artificial intelligence model.

**FIG. 1**

**FIG. 2**

**FIG. 3**

230                                          410                              330

Pair rep →

Pair rep                                                          Cross attention

← attention score

430

**FIG. 4**

Peptide                                          MHC

A          d          B

**FIG. 5**

510

**FIG. 6**

start

extracting 3D coordinate information on MHC-peptide complex — S610

extracting distance information between subunits based on 3D coordinate information — S620

learning to predict bonding of MHC-peptide complex based on distance information — S630

end

**FIG. 7**

**FIG. 8**

```
              ( start )
                 │                      S810
                 ▼
┌────────────────────────────────────────────┐
│ operating multi-head cross-attention using  │
│        peptide as query and MHC as key      │
└────────────────────────────────────────────┘
                 │                      S820
                 ▼
┌────────────────────────────────────────────┐
│  receiving pair-rep based on 3D coordinate  │
│     information of MHC-peptide complex       │
└────────────────────────────────────────────┘
                 │                      S830
                 ▼
┌────────────────────────────────────────────┐
│ summing result of cross-attention operating │
│                 and pair-rep                 │
└────────────────────────────────────────────┘
                 │
                 ▼
              ( end )
```

**FIG. 9**

```
              ( start )
                 │                      S910
                 ▼
┌────────────────────────────────────────────┐
│   receiving operation result of summing     │
│ result of cross-attention operation and     │
│                pair-rep                      │
└────────────────────────────────────────────┘
                 │                      S920
                 ▼
┌────────────────────────────────────────────┐
│  applying attention score obtained according│
│   to the operation result to initial pair-rep│
└────────────────────────────────────────────┘
                 │                      S930
                 ▼
┌────────────────────────────────────────────┐
│  predicting structure of MHC-peptide complex│
│   by pair-rep to which attention score is   │
│                 applied                      │
└────────────────────────────────────────────┘
                 │
                 ▼
              ( end )
```

**FIG. 10**

Europäisches
Patentamt

European
Patent Office

Office européen
des brevets

# EUROPEAN SEARCH REPORT

Application Number

EP 24 20 8114

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Aronson Alon ET AL: "Structure modeling and specificity of peptide-MHC class I interactions using geometric deep learning", bioRxiv, 19 December 2022 (2022-12-19), XP093259964, DOI: 10.1101/2022.12.15.520566 Retrieved from the Internet: URL:https://www.biorxiv.org/content/10.1101/2022.12.15.520566v1.full.pdf [retrieved on 2025-03-17] * the whole document * | 1-16 | INV. G16B15/30 G06N3/045 G16B40/20 G16B40/30 |
| X | US 2007/192036 A1 (JOJIC NEBOJSA [US] ET AL) 16 August 2007 (2007-08-16) | 1-3,10, 11,16 | |
| A | * paragraphs [0011], [0042] - [0044], [0049], [0052]; figures 2-8 * | 4-9, 12-15 | |
| A | US 2015/278441 A1 (MIN RENQIANG [US] ET AL) 1 October 2015 (2015-10-01) * paragraphs [0003] - [0007], [0020] - [0034]; figure 2 * | 1-16 | TECHNICAL FIELDS SEARCHED (IPC) G16B G06N |

The present search report has been drawn up for all claims

| Place of search | Date of completion of the search | Examiner |
|---|---|---|
| Munich | 17 March 2025 | Hendrikse, Natalie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 20 8114

This annex lists the patent family members relating to the patent documents cited in the above-mentioned European search report.
The members are as contained in the European Patent Office EDP file on
The European Patent Office is in no way liable for these particulars which are merely given for the purpose of information.

17-03-2025

| Patent document cited in search report | Publication date | Patent family member(s) | Publication date |
|---|---|---|---|
| US 2007192036 A1 | 16-08-2007 | US 2007192033 A1<br>US 2007192036 A1<br>US 2007192037 A1 | 16-08-2007<br>16-08-2007<br>16-08-2007 |
| US 2015278441 A1 | 01-10-2015 | NONE | |

EPO FORM P0459

For more details about this annex : see Official Journal of the European Patent Office, No. 12/82

**REFERENCES CITED IN THE DESCRIPTION**

**Patent documents cited in the description**

- KR 1020220064958